# EUROPÄISCHE PATENTSCHRIFT

(11) **EP 3 369 450 B1**
(45) Veröffentlichungstag und Bekanntmachung des Hinweises auf die Patenterteilung: **02.06.2021**
(21) Anmeldenummer: 18168709.6
(22) Anmeldetag: 15.06.2015
(51) Int. Cl.: A61M 5/32

(54) **SPRITZE MIT SICHERHEITSVORRICHTUNG**
SYRINGE WITH SAFETY DEVICE
SERINGUE AVC DISPOSITIF DE SÉCURITÉ

(43) Veröffentlichungstag der Anmeldung: 05.09.2018
(62) Teilanmeldung aus: 15172173.5
(73) Patentinhaber: Gerresheimer Regensburg GmbH, 93047 Regensburg (DE); Gerresheimer Bünde GmbH, 32257 Bünde (DE)
(72) Erfinder: WITTLAND, Frank, 32130 Enger (DE); VOGL, Maximilian, 92708 Mantel (DE)
(74) Vertreter: Hannke, Christian

(56) Entgegenhaltungen:
- WO-A1-01/54758
- WO-A1-2012/096620
- US-A- 5 591 138
- US-A1- 2014 257 200
- US-A1- 2014 364 805
- US-B1- 6 585 691

## Beschreibung

Die Erfindung betrifft eine Spritze mit einem Spritzenkörper und einer Sicherheitsvorrichtung zur Vermeidung von Stichverletzungen und einem an dem distalen Ende des Spritzenkörpers angeordneten Stechmittel, umfassend ein sich entlang einer axialen Richtung (X) erstreckendes Hülsenelement, welches zumindest teilweise das Stechmittel und den Spritzenkörper umschließt, und ein Kragenelement, welches an einem distalen Endbereich des Spritzenkörpers anordenbar ist und die Sicherheitsvorrichtung in axialer Richtung (X) arretiert, wobei das Kragenelement zumindest einen Führungsvorsprung aufweist, welcher in zumindest einer Führungskulisse des Hülsenelements eingreift.

Gattungsgemäße Spritzen mit Sicherheitsvorrichtungen zur Vermeidung von Stichverletzungen sind aus dem Stand der Technik bekannt. Insbesondere bei vorgefüllten Spritzen ist die Verwendung derartiger Sicherheitsvorrichtungen sinnvoll. Die Handhabung derartiger Spritzen ist sehr einfach, da das Medium nicht vor der Anwendung in die Spritze transferiert werden muss. Weiterhin ist, selbst im Notfall, die Wahrscheinlichkeit der Anwendung eines falschen Medikaments sehr gering. Für Impfstoffe und zahlreiche andere Medikamente sind sie heutzutage das Primärpackmittel erster Wahl. Diese Spritzen sind üblicherweise aus Glas oder Kunststoff (beispielsweise COC, COP) hergestellt und müssen mit Schutzkappen ausgestattet sein, um Beschädigungen und/oder eine Kontaminierung der Kanüle vor der Anwendung der Spritze zu vermeiden. Darüber hinaus ist es wichtig, nach Gebrauch der Spritze die Kanüle zu sichern, um Stichverletzungen zu vermeiden. Dabei kann ein unvorsichtiges Wiederaufsetzten der Schutzkappe auf die Kanüle Stichverletzungen verursachen. Oft ist die entsprechende Schutzkappe nicht mehr auffindbar oder es wird vergessen, diese wiederaufzusetzen, wodurch ein vermeidbares Verletzungsrisiko gegeben ist.

Demzufolge wurden Nadelschutzeinrichtungen entwickelt, welche fest mit der Spritze verbunden sind und die Nadel nach Verwendung der Spritze automatisch wieder aufnehmen. Eine solche Nadelschutzeinrichtung ist beispielsweise in DE 11 2009 001 083 T5 offenbart. Hierbei wird eine federangetriebene Sicherheitshülse gezeigt, welche in einem ausgefahrenen Zustand die Kanüle umgibt und diese gegen Verletzungen der Anwender sichert. Die Sicherheitshülse weist dabei eine Kurvenbahn auf, in welcher mindestens ein Führungsstift läuft, wodurch verschiedene Positionen der Sicherheitshülse in Abhängigkeit zur Nadelspitze realisiert werden können. Der mindestens eine Führungsstift muss dabei über einen Kragen an der Frontgeometrie der Spritze befestigt werden oder muss auf andere Weise fest mit der Spritze verbunden werden. Der Kragen mit dem Führungsstift darf, um Manipulation oder Fehlbenutzung vorzubeugen, nicht oder nur schwer von der Spritze mit einer Kanüle entfernbar sein. Demnach ist ein entsprechend fester Sitz in axialer Richtung notwendig.

Im Bereich der vorgefüllten Spritzen wird bereits vor dem Abfüllprozess eine Schutzkappe, beziehungsweise eine Sicherheitsvorrichtung zur Vermeidung von Stichverletzungen, auf dem Spritzenkörper montiert und in einer Standardverpackung, beispielsweise einem Spritzennest sterilisiert. Man spricht in diesem Zusammenhang auch von Ready to Use (RTU) bzw. Ready to Sterilize (RTS) Spritzen. In der Regel sollen die Sicherheitsvorrichtungen derart ausgelegt sein, dass sie leichtgängig zu betätigen sind, um einen optimalen Komfort für den Anwender zu gewährleisten. Demzufolge ist es möglich, dass die Spritzen bereits während des Transports ungewollt betätigt werden. Auch können die Spritzen versehentlich während der Benutzung ausgelöst werden. Dies kann die Spritze unter Umständen komplett unbrauchbar machen. Das beinhaltete Medikament kann nicht verabreicht werden. Überdies kann es ebenso zu Verletzungen des Anwenders oder des Patienten kommen, falls dieser die Sicherheitsvorrichtung unabsichtlich betätigt.

Aus der Druckschrift US 2014 / 257200 ist eine gattungsgemäße Spritze mit einer Nadelschutzvorrichtung bekannt. Dabei werden mehrere Ausführungsformen in dem Dokument beschrieben. Die beschriebenen Ausführungsformen umfassen einen ersten Zylinder, der am distalen Ende der Spritze angeordnet ist. Ein zweiter Zylinder ist innerhalb des ersten Zylinders angeordnet, wobei nach Nutzung der Nadel der zweite Zylinder durch ein Federelement über die Nadel verlagert wird. Gemäß einer Ausführungsform kann auch ein dritter Zylinder vorgesehen sein, welcher innerhalb des zweiten Zylinders angeordnet ist, so dass nach Nutzung der Nadel der zweite und der dritte Zylinder teleskopartig über die Nadel verlagerbar sind.

Die Druckschrift US 2014 / 0364805 A1 zeigt eine Injektorvorrichtung mit einem Gehäuse, in welchem eine Spritze angeordnet ist. An dem Gehäuse ist ein Kappenelement angeordnet, welches eine Stechmittelschutzeinrichtung umfasst. Das Kappenelement ist mit dem Gehäuse über eine Sollbruchstelle verbunden.

Die Druckschrift US 6 585 691 B1 zeigt eine Spritze mit einer manipulationssicheren Kappenvorrichtung. Die Kappenvorrichtung umfasst eine Hülse, welche über eine brechbare Verbindung mit einem Indikatorring verbunden ist.

Die Druckschrift US 5 591 138 A offenbart einen Nadelaufsatz, welcher mit einer Schutzvorrichtung ausgestattet ist. Die Schutzvorrichtung umfasst eine äußere Hülse mit einer Führungskulisse und eine innere Hülse mit einem Führungsstift, der in der Führungskulisse geführt wird. Die innere Hülse ist relativ zu dem äußeren Gehäuse verlagerbar. Innerhalb der inneren Hülse ist weiterhin ein Kappenelement angeordnet, welches die Nadel umschließt. In einer Ausgangslage ist die innere Hülse mit dem Kappenelement über der Nadel angeordnet. Zur Injektion wird die innere Hülse in die äußere Hülse geschoben. Das Kappenelement wird dabei gestaucht, so dass die Nadelspitze durch das Kappenelement tritt. Nach der Injektion wird die innere Hülse wieder über die Nadel verlagert.

Aufgabe der vorliegenden Erfindung ist es, eine Sicherheitsvorrichtung zur Vermeidung von Stichverletzungen für eine Spritze zur Verfügung zu stellen, welche die eingangs genannten Probleme löst.

Diese Aufgabe wird gelöst durch eine Sicherheitsvorrichtung zur Vermeidung von Stichverletzungen an einer Spritze mit einem Spritzenkörper und einem an dem distalen konischen Endstück des Spritzenkörpers angeordneten Stechmittel, umfassend ein sich entlang einer axialen Richtung (X) erstreckendes Hülsenelement, welches zumindest teilweise das Stechmittel und den Spritzenkörper umschließt, und ein Kragenelement, welches an einem distalen Endbereich des Spritzenkörpers anordenbar ist und die Sicherheitsvorrichtung in axialer Richtung (X) an einem Vorsprung des konischen Endstücks arretiert, wobei das Kragenelement zumindest einen Führungsvorsprung aufweist, welcher in zumindest einer Führungskulisse des Hülsenelements eingreift. Bevorzugt ist der Führungsvorsprung in der zumindest einen Führungskulisse des Hülsenelements bei einer Relativbewegung des Hülsenelements zu dem Spritzenkörper im Wesentlichen entlang der axialen Richtung (X) geführt. Die Sicherheitsvorrichtung zeichnet sich weiterhin dadurch das, dass diese ein Kappenelement aufweist, welches zumindest abschnittsweise über dem Hülsenelement anordenbar ist und durch welches das Hülsenelement bezüglich der Relativbewegung des Spritzenkörpers zu dem Hülsenelement arretierbar ist.

Da durch das Kappenelement das Hülsenelement bezüglich der Relativbewegung des Spritzenkörpers zu dem Hülsenelement arretierbar ist, wird effektiv ein unerwünschtes Hervortreten des Stechmittels, welches eine Kanüle, eine Nadel oder auch eine Lanzette sein kann, durch eine entsprechende Öffnung in der Sicherheitsvorrichtung unterbunden. Beschädigungen und Kontamination des Stechmittels werden demnach verhindert. Der Anwender der Spritze muss zunächst das Kappenelement von dem Hülsenelement abziehen, bevor die Spritze angewendet werden kann. Demnach wird ebenso das Risiko eines versehentlichen Betätigens verringert. Es wäre denkbar, an dem Kappenelement eine Markierung oder einen Hinweis anzubringen. Der Anwender wäre somit gezwungen, diese Markierung, beziehungsweise den Hinweis vor der Anwendung der Spritze, wahrzunehmen. Eine derartige Markierung, beziehungsweise ein derartiger Hinweis, könnte farblich und/oder haptisch und/oder anderweitig ausgestaltet sein.

Gemäß einer besonders bevorzugten Ausführungsform ist das Kragenelement im Wesentlichen als hohler Kreiszylinder ausgebildet. Bevorzugt weist der Kreiszylinder eine Mantelfläche auf, an der der zumindest eine Führungsvorsprung angeordnet ist. Vorzugsweise erstreckt sich der zumindest eine Führungsvorsprung radial von der Mantelfläche weg. Weiterhin bevorzugt ist der Führungsvorsprung als Kreiszylinder, beziehungsweise als Stift ausgebildet. Vorteilhafterweise sind an der Mantelfläche zwei sich diametral gegenüberliegende Führungsvorsprünge angeordnet. Demzufolge würde auch das Hülsenelement zwei sich diametral gegenüberliegende Führungskulissen aufweisen, in denen jeweils ein Führungsvorsprung geführt wird. Vorzugsweise ist das Kragenelement weiterhin in einer Umfangsrichtung (U) rotierbar an dem distalen Endbereich des Spritzenkörpers angeordnet. Bei einer Anwendung der Spritze wird die Spritze mit der Sicherheitseinrichtung gegen die Haut des Patienten gedrückt. Durch die Relativbewegung des Spritzenkörpers zu dem Hülsenelement und die Führung des Führungsvorsprungs in der Führungskulisse wird eine Rotation des Kragenelements entlang einer Umfangsrichtung (U) verursacht. Das Hülsenelement schiebt sich dadurch vorzugsweise über den Spritzenkörper, wodurch das Stechmittel, welches eine Kanüle, eine Nadel oder auch eine Lanzette sein kann, durch eine entsprechende Öffnung in dem Hülsenelement hindurchtritt. Somit wird eine Rotation des Hülsenelements auf der Haut des Patienten um die Einstichstelle herum vermieden.

Vorzugsweise ist der Spritzenkörper als hohler Kreiszylinder ausgestaltet und weist in seinem distalen Endbereich ein konisches Endstück auf, an welchem das Stechmittel angeordnet ist. Bevorzugt ist an dem konischen Endstück ein Vorsprung ausgebildet, an welchem eine Stirnfläche des distalen Endes des Kragenelements eingreifbar ist, wodurch das Kragenelement und somit die Sicherheitsvorrichtung in axialer Richtung arretierbar ist. Weiterhin bevorzugt ist auch die Sicherheitsvorrichtung im Wesentlichen in Form eines hohlen Kreiszylinders ausgestaltet.

Gemäß der Erfindung weist das Kappenelement eine Stechmittelschutzeinrichtung auf, in welcher das Stechmittel anordenbar ist. Durch eine derartige Stechmittelschutzeinrichtung wird ein weiterer Schutz des Stechmittels vor Beschädigungen und insbesondere vor Kontamination gewährleistet.

Vorzugsweise weist das Hülsenelement eine distale Öffnung auf. Bevorzugt ist dabei der Innendurchmesser der distalen Öffnung zumindest abschnittsweise größer als der Außendurchmesser der Stechmittelschutzeinrichtung, so dass die Stechmittelschutzeinrichtung innerhalb des Hülsenelements anordenbar ist.

Die Stechmittelschutzeinrichtung ist mit dem Kragenelement in Wirkkontakt bringbar, wodurch das Kragenelement bezüglich einer Rotation arretierbar ist. Ein solcher Wirkkontakt kann beispielweise ein reibschlüssiger Kontakt sein. Es wäre aber auch denkbar, dass die Stechmittelschutzeinrichtung und das Kragenelement zueinander korrespondierende Rasteinrichtungen aufweisen, welche eine Rotation des Kragenelements verhindern.

Gemäß der Erfindung weisen das Kappenelement und das Hülsenelement zueinander komplementäre Rastelemente auf, so dass das Kappenelement und das Hülsenelement trennbar verrastbar sind. Es wäre vorstellbar, dass ein Rastelement eine Sollbruchstelle aufweist, welche vor der Anwendung aufgebrochen werden muss, um ein Abziehen des Kappenelements von dem Hülsenelement zu ermöglichen. Es wäre aber auch denkbar, dass ein Einrasten der Rastelemente auch nach der Anwendung der Spritze ermöglicht ist. Somit wäre es ermöglicht, dass Kappenelement nach dem Gebrauch der Spritze wieder fest auf dem Hülsenelement anzuordnen, wodurch das Hülsenelement bezüglich der Relativbewegung des Spritzenkörpers zu dem Hülsenelement wiederum arretierbar wäre. Demzufolge ist eine gefahrlose Entsorgung der gebrauchten Spritze möglich, welche somit kein Verletzungsrisiko mehr darstellt.

Gemäß eines weiteren bevorzugten Gedankens der Erfindung umfasst das Kappenelement zumindest ein flügelartiges Element, welches in zumindest einer Aufnahme des Hülsenelements aufnehmbar ist. Weiterhin bevorzugt weist das Kappenelement zwei flügelartige Elemente auf, welche diametral in jeweils zwei Aufnahmen des Hülsenelements aufnehmbar sind. Besonders bevorzugt sind die beiden flügelartigen Elemente diametral sich gegenüberliegend an dem Kappenelement angeordnet.

Nach einer weiteren bevorzugten Ausführungsform ist zumindest ein flügelartiges Element mit dem Kragenelement in Wirkkontakt bringbar, wodurch das Kragenelement bezüglich einer Rotation arretierbar ist. Ein solcher Wirkkontakt kann beispielweise ein reibschlüssiger Kontakt sein. Es wäre aber auch denkbar, dass die Stechmittelschutzeinrichtung und das Kragenelement zueinander korrespondierende Rasteinrichtungen aufweisen, welche eine Rotation des Kragenelements verhindern.

Gemäß einer weiteren bevorzugten Ausführungsform ist an dem zumindest einen flügelartigen Element des Kappenelements zumindest ein Rastelement angeordnet, welches in zumindest ein komplementäres Rastelement, das in der zumindest einen Aufnahme des Hülsenelements angeordnet ist, einrastbar ist.

Es wäre auch denkbar, dass das Hülsenelement einen distalen Bereich aufweist, an welchem zumindest ein Rastelement angeordnet ist. Vorteilhafterweise ist das zumindest eine Rastelement in zumindest ein komplementäres Rastelement einrastbar, welches in einem distalen Bereich des Kappenelements angeordnet ist.

Vorzugsweise ist das Kappenelement integral mit der Stechmittelschutzeinrichtung ausgebildet. Eine derartige Ausführung der Sicherheitsvorrichtung hat eine kostengünstige und einfache Herstellung zum Vorteil.

Es ist aber auch denkbar, dass das Kappenelement eine distale Öffnung aufweist, wobei die distale Öffnung als Aufnahme ausgebildet ist, um die Stechmittelschutzeinrichtung aufzunehmen. Eine solche Ausgestaltung ermöglicht es, das Kappenelement und die Stechmittelschutzeinrichtung aus unterschiedlichen Materialien herzustellen. Es wäre demnach denkbar, die Stechmittelschutzeinrichtung aus einem elastischen Material, beispielsweise Gummi herzustellen. Ein solches elastisches Material begünstigt eine Reduzierung des Beschädigungsrisikos des Stechmittels.

Vorzugsweise weist die Sicherheitsvorrichtung mindestens ein Federelement auf, das mit dem Spritzenkörper wirkverbunden ist und der Relativbewegung des Spritzenkörpers zu der Sicherheitsvorrichtung entgegenwirkt. Demnach bleibt die Kanüle bis zur vorgesehenen Anwendung innerhalb des Hülsenelements. Bei der Anwendung muss das Hülsenelement gegen die Federkraft verschoben werden, damit die Kanüle durch die Öffnung des Hülsenelements hindurchtreten kann. Nach Gebrauch der Spritze schiebt sich automatisch, angetrieben durch die Federkraft des Federelements, das Hülsenelement wieder über die Kanüle. Durch die Führung des Führungsvorsprungs in der Führungskulisse rotiert das Kragenelement entgegen der Umfangsrichtung (U). Der Anwender ist somit vor Stichverletzungen mit der gebrauchten kontaminierten Kanüle geschützt. Bevorzugt umfasst das Federelement eine Spiralfeder. Denkbar sind aber auch anderweitige Federarten, wie beispielsweise Schenkelfedern oder Torsionsfedern. Ferner wäre vorstellbar, das Federelement als ein Elastomer auszubilden.

Nach einem weiteren vorteilhaften Gedanken der Erfindung umfasst die zumindest eine Führungskulisse einen ersten und einen zweiten Kulissenbereich, die durch eine entlang der axialen Richtung (X) des Spritzenkörpers verlaufende fiktive Trennlinie voneinander getrennt sind, wobei der Führungsvorsprung in einer Ausgangsstellung in dem ersten Kulissenbereich anordenbar ist und von dem ersten in den zweiten Kulissenbereich in eine Endstellung durch Überschreiten der Trennlinie überführbar ist, wenn ein distales Ende des Stechelements bei der Relativbewegung von Spritzenkörper zu Hülsenelement auf der Höhe der distalen Öffnung des Hülsenelements angeordnet ist.

Demnach ist der Führungsvorsprung des Kragenelements von dem ersten Kulissenbereich in den zweiten Kulissenbereich überführbar. Diese Überführung findet statt, wenn der Führungsvorsprung eine fiktive Trennlinie, welche den ersten und den zweiten Kulissenbereich voneinander trennt, überschreitet. Befindet sich der Führungsvorsprung in dem ersten Kulissenbereich, also in einer Ausgangsstellung, so wurde die Spritze noch nicht ausgelöst, d.h. das Stechmittel hat die Sicherheitsvorrichtung noch nicht verlassen. Befindet sich der Führungsvorsprung in dem zweiten Kulissenbereich, so ist das Stechmittel aus der Sicherheitsvorrichtung bereits ausgetreten, so dass eine Injektion möglich ist. Beim Übergang von dem ersten Kulissenbereich zu dem zweiten Kulissenbereich, also genau dann, wenn der Führungsvorsprung die Trennlinie überschreitet, befindet sich das distale Ende des Stechmittels auf der Höhe der distalen Öffnung der Sicherheitsvorrichtung.

Bevorzugt ist der Führungsvorsprung von dem zweiten Kulissenbereich mittels einer Kulisse des zweiten Kulissenbereichs in einen Endbereich überführbar, in welchem eine Relativbewegung des Hülsenelements zu dem Spritzenkörper im Wesentlichen entlang der axialen Richtung (X) zumindest eingeschränkt ist. Durch eine derartige Ausgestaltung ist ein weiteres Verschieben des Hülsenelements relativ zum Spritzenkörper zumindest eingeschränkt, bevorzugt verhindert. Demzufolge ist ein weiteres Austreten des Stechmittels aus der Sicherheitsvorrichtung nach der Anwendung der Spritze unterbunden.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
Fig.1 eine Schnittdarstellung einer Spritze mit Sicherheitsvorrichtung gemäß einer Ausführungsform;
Fig.2 eine Schnittdarstellung einer Spritze mit Sicherheitsvorrichtung gemäß einer weiteren Ausführungsform;
Fig.3 eine Schnittdarstellung einer Spritze mit Sicherheitsvorrichtung gemäß einer weiteren Ausführungsform;
Fig.4 eine Schnittdarstellung einer Spritze mit Sicherheitsvorrichtung gemäß einer weiteren Ausführungsform;
Fig.5 eine isometrische Ansicht eines Hülsenelements gemäß einer weiteren Ausführungsform;
Fig.6 eine isometrische Ansicht eines Hülsenelements gemäß einer weiteren Ausführungsform;
Fig.7 eine isometrische Ansicht eines Kappenelements gemäß einer weiteren Ausführungsform;
Fig.8 eine Seitenansicht einer an einem Spritzenkörper angeordneten Sicherheitsvorrichtung.

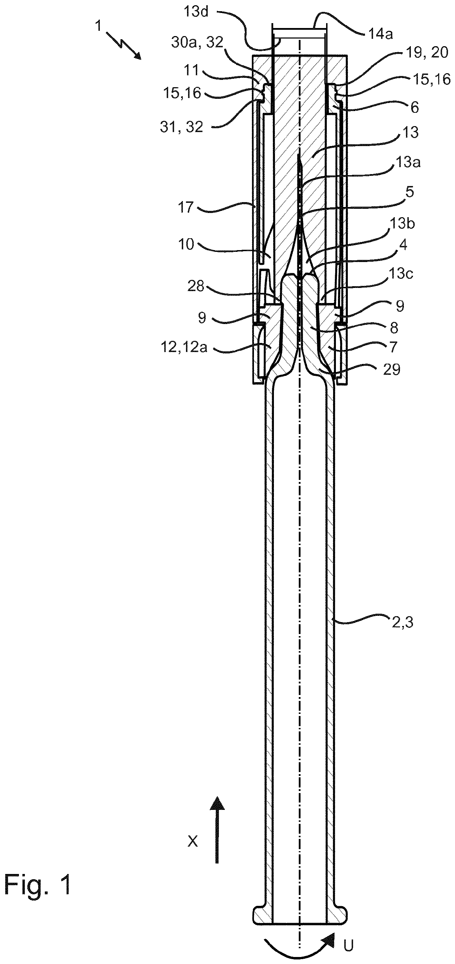

In den Figuren 1 bis 4 ist eine Spritze (2) mit einer Sicherheitsvorrichtung (1) zur Vermeidung von Stichverletzungen gemäß unterschiedlicher Ausführungsformen gezeigt. Die Spritze (2) umfasst einen Spritzenkörper (3), welcher als hohler Kreiszylinder ausgestaltet ist. Der Spritzenkörper weist einen distalen Endbereich (8) mit einem distalen Ende (4) auf. An dem distalen Ende (4) ist ein Stechmittel (5) angeordnet. Dieses Stechmittel (5) ist über eine Bohrung in dem distalen Endbereich (8) mit dem Hohlraum des Spritzenkörpers (3) verbunden, so dass das zu injizierende Medium bei einer Anwendung der Spritze (2) aus dem Hohlraum durch das Stechmittel (5) treten kann. Der distale Endbereich (8) ist als konisches Endstück ausgestaltet, welches einen kleineren Außendurchmesser als der Spritzenkörper (3) aufweist. Weiterhin weist die Spritze einen Übergangsbereich (29) auf, in dem der Außendurchmesser des Spritzenkörpers in den Außendurchmesser des Endstücks übergeht. Darüber hinaus ist an dem distalen Endbereich ein Vorsprung (28) angeordnet.

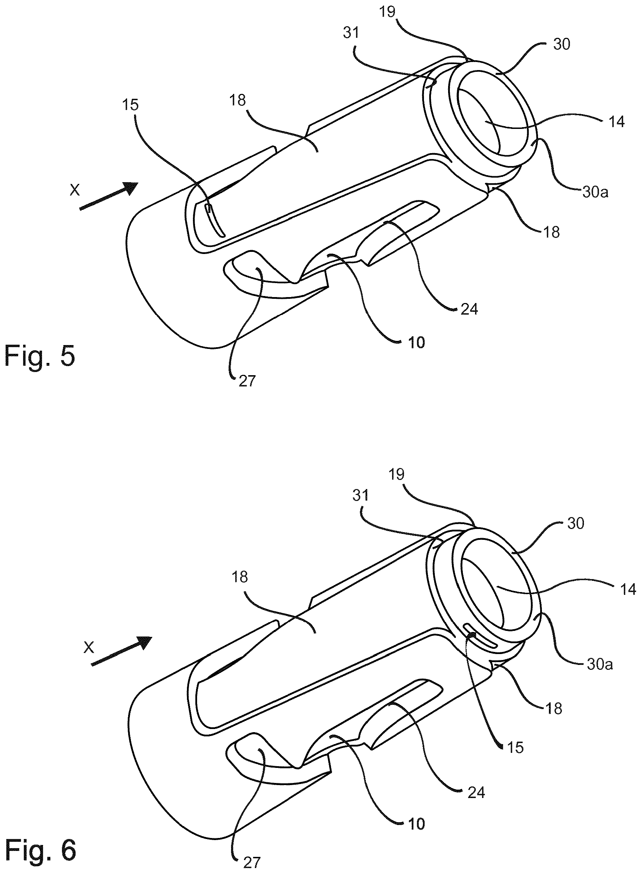

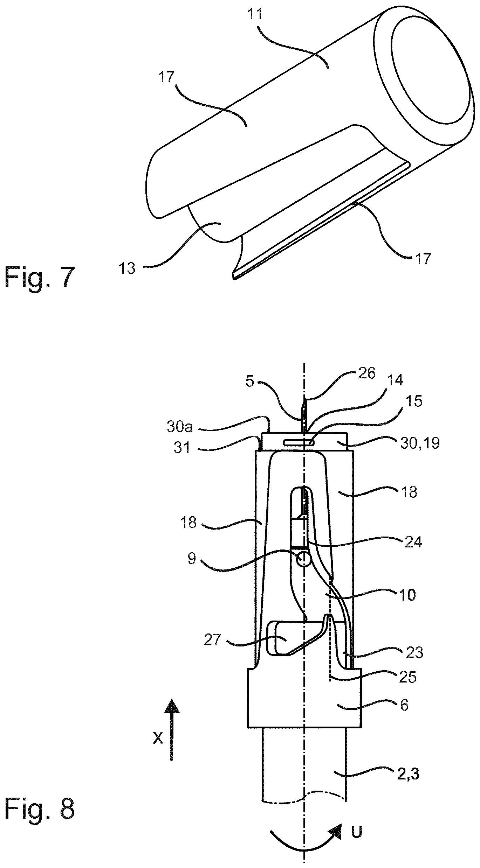

Weiterhin ist eine Sicherheitsvorrichtung (1) zur Vermeidung von Stichverletzungen für eine Spritze (2) gezeigt, welche einen Spritzenkörper (3) und ein an dem distalen Ende (4) des Spritzenkörpers (3) angeordnetes Stechmittel (5) umfasst. Die Sicherheitsvorrichtung (1) umfasst ein sich entlang einer axialen Richtung (X) erstreckendes Hülsenelement (6), welches zumindest teilweise das Stechmittel (5) und den Spritzenkörper (3) umschließt, und ein Kragenelement (7), welches an einem distalen Endbereich (8) des Spritzenkörpers (3) angeordnet ist und die Sicherheitsvorrichtung (1) in axialer Richtung (X) arretiert. Ferner weist die Sicherheitsvorrichtung (1) ein Kappenelement (11) auf, welches zumindest abschnittsweise über dem Hülsenelement (6) anordenbar ist und durch welches das Hülsenelement (6) bezüglich der Relativbewegung des Spritzenkörpers (3) zu dem Hülsenelement (6) arretierbar ist. Das Hülsenelement (6) und das Kappenelement (11) sind dabei im Wesentlichen zylindrisch ausgestaltet. Das Hülsenelement (6) wird weiterhin in den Figuren 5 und 6 detaillier in einer isometrischen Ansicht dargestellt. In Fig. 8 ist eine Seitenansicht des Hülsenelements (6) gezeigt, wobei dieses an dem Spritzenkörper (3) angeordnet ist. Das Kappenelement (11) ist in Fig. 7 detailliert in einer isometrischen Ansicht dargestellt.

Die Arretierung in axialer Richtung wird durch einen Vorsprung (28) bzw. eine Verdickung an dem distalen Ende (4) des Spritzenkörpers ermöglicht, an welchem das Kragenelement (7) mit seinem distalen Ende anliegt.

Das Kragenelement (7) ist im Wesentlichen als hohler Kreiszylinder (12) ausgebildet. Der Kreiszylinder (12) weist eine Mantelfläche (12a) auf, an der zwei Führungsvorsprünge (9) angeordnet sind. Die Führungsvorsprünge (9) erstrecken sich radial von der Mantelfläche (12a) nach außen weg und sind diametral gegenüberliegend angeordnet. Weiterhin sind diese als Kreiszylinder, beziehungsweise als Stift, ausgebildet. Diese beiden Führungsvorsprünge (9) sind jeweils in einer Führungskulisse (10) des Hülsenelements (6) bei einer Relativbewegung des Spritzenkörpers (3) zu dem Hülsenelement (6) im Wesentlichen entlang der axialen Richtung (X) geführt. Ferner ist das Kragenelement (7) in einer Umfangsrichtung (U) rotierbar an dem distalen Endbereich (8) des Spritzenkörpers (3) angeordnet.

Das Kappenelement (11) weist eine Stechmittelschutzeinrichtung (13) auf, in welcher das Stechmittel (5) anordenbar ist. Darüber hinaus umfasst das Hülsenelement (6) eine distale Öffnung (14), wobei der Innendurchmesser (14a) dieser distalen Öffnung (14) größer ist als der Außendurchmesser (13d) der Stechmittelschutzeinrichtung (13), so dass die Stechmittelschutzeinrichtung (13) innerhalb des Hülsenelements (6) anordenbar ist.

Das distale Ende (26) und ein anschließender distaler Bereich des Stechmittels (5) sind dabei in einem Hohlraum der Stechmittelschutzeinrichtung (13) angeordnet. Der Hohlraum umfasst einen ersten Bereich (13a), wobei die Stechmittelschutzeinrichtung (13) in diesem ersten Bereich an dessen Innenwänden anliegend ist. In einem dritten Bereich (13c) der Stechmittelschutzeinrichtung (13) erstreckt sich der Hohlraum weiterhin über das distale Ende (4) des Spritzenkörpers. Zwischen dem ersten (13a) und dem dritten Bereich (13c) befindet sich ein zweiter Bereich (13b), in dem sich der Innendurchmesser des Hohlraums ausgehend von dem ersten Bereich (13a) bis zu dem dritten Bereich (13c) vergrößert. Die Stechmittelschutzeinrichtung (13) erstreckt sich über den distalen Endbereich (8) des Spritzenkörpers (3) bis zu dem Kragenelement (7). Die Stechmittelschutzeinrichtung (13) ist mit dem Kragenelement (7) in Wirkkontakt, wodurch das Kragenelement (7) bezüglich einer Rotation arretierbar ist.

Das Kappenelement (11) umfasst weiterhin zwei diametral gegenüberliegende flügelartige Elemente (17). Dies ist auch in Fig. 7 zu erkennen. Diese flügelartigen Elemente (17) sind komplementär zu Aufnahmen (18) des Hülsenelements (6) ausgestaltet. Die Aufnahmen sind in Form von Ausnehmungen in dem Hülsenelement (6) ausgestaltet. Dies ist auch in den Figuren 5 und 6 zu erkennen. Wird das Kappenelement (11) auf dem Hülsenelement (6) angeordnet, so sind die beiden flügelartigen Elemente (17) in den beiden Aufnahmen (18) des Hülsenelements (6) aufnehmbar. Die Stechmittelschutzeinrichtung (13) erstreckt sich dabei durch die distale Öffnung (14) des Hülsenelements (6). Denkbar wäre auch, dass zumindest ein flügelartiges Element (17) mit dem Kragenelement (7) in Wirkkontakt ist, wodurch das Kragenelement (7) bezüglich einer Rotation arretierbar ist.

Der distale Bereich (19) des Hülsenelements (6) umfasst die distale Öffnung (14) des Hülsenelements (6) und einen Kreisring (30), welcher die distale Öffnung (14) des Hülsenelements (6) umrandet. Der Außendurchmesser des Kreisrings (30) ist dabei kleiner als der Außendurchmesser des anschließenden Bereichs des Hülsenelements (6). Somit sind an dem Hülsenelement (6) eine erste (30a) und eine zweite Stirnfläche (31) ausgebildet. Das Kappenelement (11) ist dementsprechend komplementär ausgebildet, so dass dieses in seinem distalen Bereich (20) innenseitig Auflageflächen (32) aufweist, welche an den beiden Stirnflächen (30a, 31) anliegen.

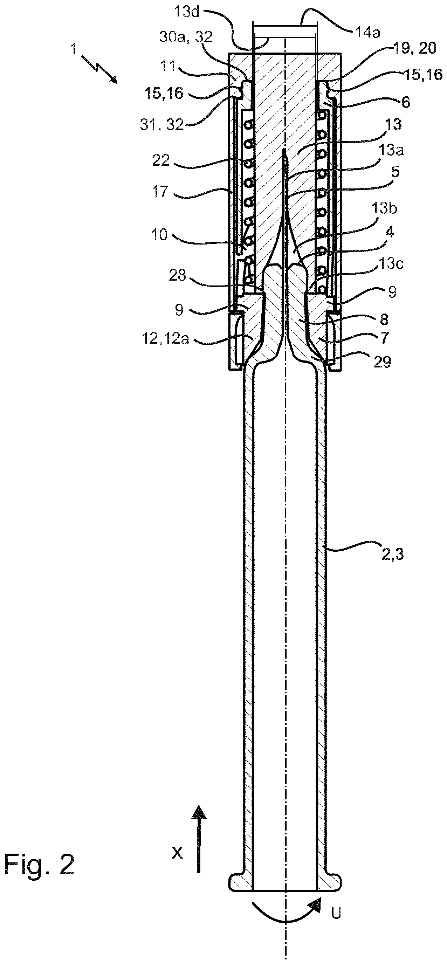

In den Figuren 1 und 2 sind Ausführungsformen der Sicherheitsvorrichtung (1) gezeigt, in denen das Kappenelement (11) integral mit der Stechmittelschutzeinrichtung (13) ausgebildet ist.

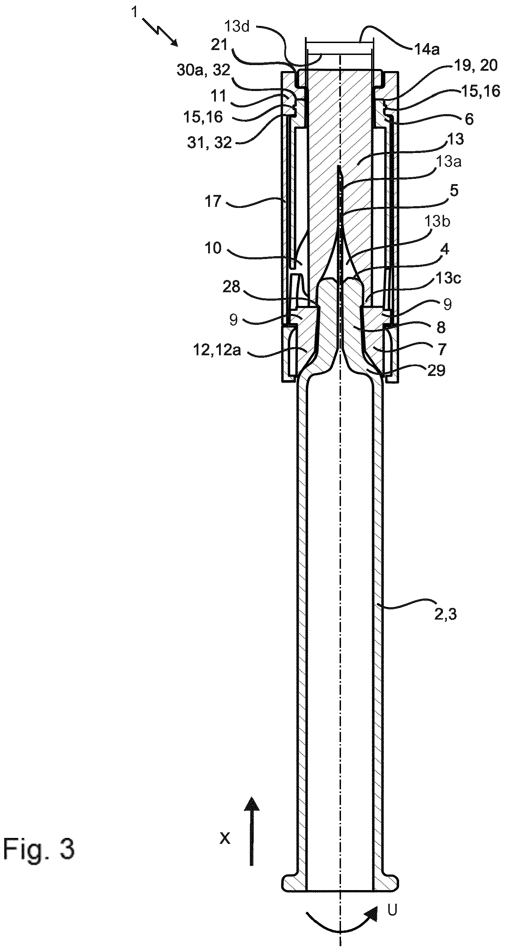

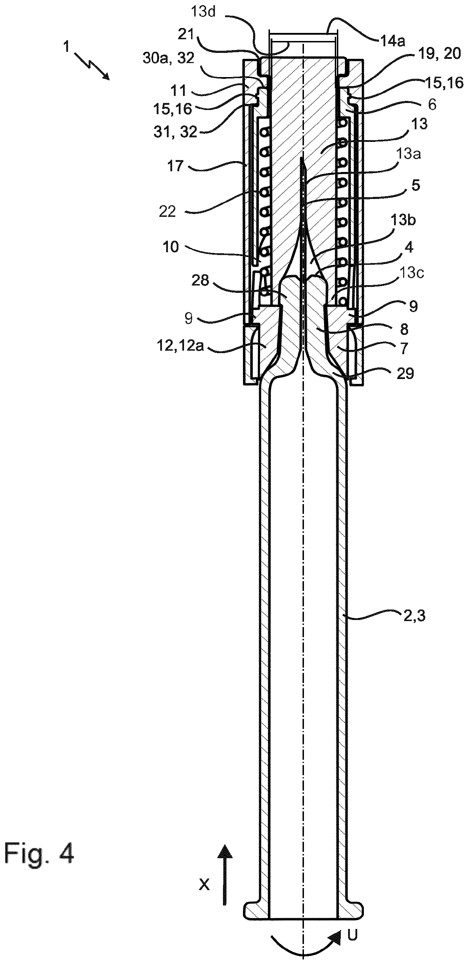

In den Figuren 3 und 4 sind Ausführungsformen der Sicherheitsvorrichtung (1) gezeigt, in denen das Kappenelement (11) eine distale Öffnung (21) aufweist. Die distale Öffnung (21) ist dabei als Aufnahme ausgebildet, um die Stechmittelschutzeinrichtung (13) aufzunehmen. Die Stechmittelschutzeinrichtung (13) umfasst dabei einen Flansch an dessen distalen Ende, welcher in der Aufnahme der distalen Öffnung (21) des Kappenelements (11) eingebettet ist.

In Fig. 2 und Fig. 4 ist weiterhin eine Sicherheitsvorrichtung (1) dargestellt, welche ein Federelement (22) in Form einer Spiralfeder aufweist, das mit dem Spritzenkörper (3) wirkverbunden ist und der Relativbewegung des Hülsenelements (6) zu der Sicherheitsvorrichtung (1) entgegenwirkt. Demnach bleibt das Stechmittel (5) bis zur vorgesehenen Anwendung innerhalb des Hülsenelements (6). Bei der Anwendung muss das Hülsenelement (6) gegen die Federkraft verschoben werden, damit das Stechmittel (5) durch die distale Öffnung (14) des Hülsenelements (6) hindurchtreten kann. Nach Gebrauch der Spritze (2) schiebt sich automatisch, angetrieben durch die Federkraft des Federelements (22), das Hülsenelement (6) wieder über das Stechmittel (5). Durch die Führung der Führungsvorsprünge (9) in den Führungskulissen (10) rotiert das Kragenelement (7) in entgegen der Umfangsrichtung (U). Der Anwender ist somit vor Stichverletzungen mit dem gebrauchten und kontaminierten Stechmittel geschützt.

Gemäß den Ausführungsformen, gezeigt in den Fig. 1 bis 4 und 6, weisen das Kappenelement (11) und das Hülsenelement (6) zueinander komplementäre Rastelemente (15, 16) auf, so dass das Kappenelement (11) und das Hülsenelement (6) trennbar verrastbar sind. Bei den vorliegenden Ausführungsformen umfasst das Hülsenelement (6) einen distalen Bereich (19), an welchem Rastelemente (15) angeordnet sind. Diese Rastelemente (15) sind in komplementäre Rastelemente (16) einrastbar, welche in einem distalen Bereich (20) des Kappenelements (11) angeordnet sind. Insbesondere sind die Rastelemente (15) des Hülsenelements (6) an einer Mantelfläche des Kreisrings (30) angeordnet.

In Fig. 5 ist ein Hülsenelement (6) dargestellt, welches zwei Aufnahmen (18) für die flügelartigen Elemente (17) des Kappenelements (11) aufweist. In den Aufnahmen (18) sind Rastelemente (15) angeordnet, welche komplementär zu entsprechenden Rastelementen (16) der flügelartigen Elemente (17) des Kappenelements (11) (hier nicht gezeigt) ausgebildet sind und in diese einrastbar sind.

Die Führungskulissen (10) des Hülsenelements (6) umfassen einen ersten (23) und einen zweiten Kulissenbereich (24), welche durch eine entlang des axialen Richtung (X) des Spritzenkörpers (3) verlaufenden fiktiven Trennlinie (25) voneinander getrennt sind, wobei der Führungsvorsprung (9) in einer Ausgangsstellung in dem ersten Kulissenbereich (23) anordenbar ist und von dem ersten (23) in den zweiten Kulissenbereich (24) in eine Endstellung durch Überschreiten der Trennlinie (25) überführbar ist, wenn ein distales Ende (26) des Stechmittels (5) bei der Relativbewegung von Spritzenkörper (3) zu Hülsenelement (6) auf der Höhe der distalen Öffnung (14) des Hülsenelements (6) angeordnet ist. Dies ist in den Figuren 5, 6 und 8 zu erkennen, wobei in Fig. 8 der Führungsvorsprung (9) die Trennlinie (25) überschritten hat, wodurch das distale Ende (26) des Stechmittels (5) bereits über die distale Öffnung (14) des Hülsenelements (6) hinausragt.

Ferner weist das Hülsenelement (6) einen Endbereich (27) auf. Die Führungsvorsprünge (9) sind dabei von dem zweiten Kulissenbereich (24) mittels einer Kulisse des zweiten Kulissenbereichs (24) in einen Endbereich (27) überführbar. In diesem Endbereich (27) ist eine Relativbewegung des Hülsenelements (6) zu dem Spritzenkörper (3) im Wesentlichen entlang der axialen Richtung (X) zumindest eingeschränkt.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

1 Sicherheitsvorrichtung
2 Spritze
3 Spritzenkörper
4 distales Ende des Spritzenkörpers
5 Stechmittel
6 Hülsenelement
7 Kragenelement
8 distaler Endbereich des Spritzenkörpers
9 Führungsvorsprung
10 Führungskulisse
11 Kappenelement
12 hohler Kreiszylinder
12a Mantelfläche des Kreiszylinders
13 Stechmittelschutzeinrichtung
13a erster Bereich
13b zweiter Bereich
13c dritter Bereich
13d Außendurchmesser
14 distale Öffnung des Hülsenelements
14a Innendurchmesser
15 Rastelement
16 Rastelement
17 flügelartiges Element
18 Aufnahme
19 distalen Bereich des Hülsenelements
20 distalen Bereich des Kappenelements
21 distale Öffnung des Kappenelements
22 Federelement
23 erster Kulissenbereich
24 zweiter Kulissenbereich
25 Trennlinie
26 distales Ende des Stechmittels
27 Endbereich
28 Vorsprung
29 Übergangsbereich
30 Kreisring
30a erste Stirnfläche
31 zweite Stirnfläche
32 Auflageflächen
X axiale Richtung
U Umfangsrichtung

## Patentansprüche

1. Spritze (2) mit einem Spritzenkörper (3), einem an dem distalen konischen Endstück (4) des Spritzenkörpers (3) angeordneten Stechmittel (5) und eine Sicherheitsvorrichtung (1) zur Vermeidung von Stichverletzungen, umfassend ein sich entlang einer axialen Richtung (X) erstreckendes Hülsenelement (6), welches zumindest teilweise das Stechmittel (5) und den Spritzenkörper (3) umschließt, und ein Kragenelement (7), welches an einem distalen Endbereich (8) des Spritzenkörpers (3) anordenbar ist und die Sicherheitsvorrichtung (1) in axialer Richtung (X) an einem Vorsprung des konischen Endstücks arretiert, wobei das Kragenelement (7) zumindest einen Führungsvorsprung (9) aufweist, welcher in zumindest einer Führungskulisse (10) des Hülsenelements (6) eingreift,
**dadurch gekennzeichnet, dass**
das Kragenelement (7) in einer Umfangsrichtung (U) rotierbar an dem distalen Endbereich (8) des Spritzenkörpers (3) angeordnet ist und die Sicherheitsvorrichtung (1) ein zumindest abschnittsweise über dem Hülsenelement (6) anordenbares Kappenelement (11) aufweist, durch welches das Hülsenelement (6) bezüglich der Relativbewegung des Spritzenkörpers (3) zu dem Hülsenelement (6) arretierbar ist, und welches eine Stechmittelschutzeinrichtung (13) aufweist, in der das Stechmittel (5) anordenbar ist, wobei das Kappenelement (11) und das Hülsenelement (6) zueinander komplementäre Rastelemente (15, 16) aufweisen, wobei ein Rastelement (15, 16) eine Sollbruchstelle aufweist und wobei ein Abziehen des Kappenelementes (11) von dem Hülsenelement (6) ein Aufbrechen der Sollbruchstelle erfordert.

2. Spritze (2) nach Anspruch 1,
**dadurch gekennzeichnet, dass**
das Kragenelement (7) im Wesentlichen als hohler Kreiszylinder (12) ausgebildet ist, wobei der Kreiszylinder (12) eine Mantelfläche (12a) aufweist, an der der zumindest eine Führungsvorsprung (9) angeordnet ist.

3. Spritze (2) nach Anspruch 1,
**dadurch gekennzeichnet, dass**
eine Rotation des Kragenelements (7) durch die Relativbewegung des Spritzenkörpers (3) zu dem Hülsenelement (6) und durch die Führung des Führungsvorsprungs (9) in der Führungskulisse (10) verursacht ist.

4. Spritze (2) nach Anspruch 1,
**dadurch gekennzeichnet, dass**
das Hülsenelement (6) einen distalen Bereich (19) aufweist, an welchem zumindest ein Rastelement (15) angeordnet ist.

5. Spritze (2) nach Anspruch 3,
**dadurch gekennzeichnet, dass**
das Hülsenelement (6) eine distale Öffnung (14) aufweist, wobei der Innendurchmesser (14a) der distalen Öffnung (14) zumindest abschnittsweise größer ist als der Außendurchmesser (13d) der Stechmittelschutzeinrichtung (13), so dass die Stechmittelschutzeinrichtung (13) innerhalb des Hülsenelements (6) anordenbar ist.

6. Spritze (2) nach Anspruch 3 oder 4,
**dadurch gekennzeichnet, dass**
die Stechmittelschutzeinrichtung (13) mit dem Kragenelement (7) in Wirkkontakt bringbar ist, wodurch das Kragenelement (7) bezüglich einer Rotation arretierbar ist.

7. Spritze (2) nach Anspruch 3 - 6
**dadurch gekennzeichnet, dass**
das Kappenelement (11) integral mit der Stechmittelschutzeinrichtung (13) ausgebildet ist.

8. Spritze (2) nach Anspruch 3 - 6
**dadurch gekennzeichnet, dass**
das Kappenelement (11) eine distale Öffnung (21) aufweist, wobei die distale Öffnung (21) als Aufnahme ausgebildet ist, um die Stechmittelschutzeinrichtung (13) aufzunehmen.

9. Spritze (2) nach einem der vorhergehenden Ansprüche,
**dadurch gekennzeichnet, dass**
die Sicherheitsvorrichtung (1) mindestens ein Federelement (22) aufweist, das mit dem Spritzenkörper (3) wirkverbunden ist und der Relativbewegung des Hülsenelements (6) zu der Sicherheitsvorrichtung (1) entgegenwirkt.

10. Spritze (2) nach einem der vorhergehenden Ansprüche,
**dadurch gekennzeichnet, dass**
die zumindest eine Führungskulisse (10) einen ersten (23) und einen zweiten Kulissenbereich (24) umfasst, welche durch eine entlang des axialen Richtung (X) des Spritzenkörpers (3) verlaufende fiktive Trennlinie (25) voneinander getrennt sind, wobei der Führungsvorsprung (9) in einer Ausgangsstellung in dem ersten Kulissenbereich (23) anordenbar ist und von dem ersten (23) in den zweiten Kulissenbereich (24) in eine Endstellung durch Überschreiten der Trennlinie (25) überführbar ist, wenn ein distales Ende (26) des Stechmittels (5) bei der Relativbewegung von Spritzenkörper (3) zu dem Hülsenelement (6) auf der Höhe der distalen Öffnung (14) des Hülsenelements (6) angeordnet ist.

11. Spritze (2) nach Anspruch 10,
**dadurch gekennzeichnet, dass**
der zumindest eine Führungsvorsprung (9) von dem zweiten Kulissenbereich (24) mittels einer Kulisse des zweiten Kulissenbereichs (24) in einen Endbereich (27) überführbar ist, in welchem eine Relativbewegung des Hülsenelements (6) zu dem Spritzenkörper (3) im Wesentlichen entlang der axialen Richtung (X) zumindest eingeschränkt ist.

## Claims

1. Syringe (2) comprising a syringe body (3), a piercing means (5) arranged on the distal conical end piece (4) of the syringe body (3), and a safety device (1) for preventing puncture wounds, comprising a sleeve element (6) which extends along an axial direction (X) and at least partially encloses the piercing means (5) and the syringe body (3), and a collar element (7) which can be arranged on a distal end region (8) of the syringe body (3) and locks the safety device (1) in the axial direction (X) on a projection of the conical end piece, the collar element (7) having at least one guide projection (9) which engages in at least one guide slot (10) of the sleeve element (6), **characterised in that** the collar element (7) is arranged on the distal end region (8) of the syringe body (3) so as to be rotatable in a circumferential direction (U), and the safety device (1) has a cap element (11) which can be arranged over the sleeve element (6) at least in portions and by means of which the sleeve element (6) can be locked with respect to the movement of the syringe body (3) relative to the sleeve element (6) and which has a piercing means protection device (13) in which the piercing means (5) can be arranged, the cap element (11) and the sleeve element (6) having latching elements (15, 16) which are complementary to one another, a latching element (15, 16) having a predetermined breaking point, and removal of the cap element (11) from the sleeve element (6) requiring the predetermined breaking point to be broken.

2. Syringe (2) according to claim 1, **characterised in that** the collar element (7) is substantially formed as a hollow circular cylinder (12), the circular cylinder (12) having a lateral surface (12a) on which the at least one guide projection (9) is arranged.

3. Syringe (2) according to claim 1, **characterised in that** a rotation of the collar element (7) is caused by the movement of the syringe body (3) relative to the sleeve element (6) and by the guidance of the guide projection (9) in the guide slot (10).

4. Syringe (2) according to claim 1, **characterised in that** the sleeve element (6) has a distal region (19) on which at least one latching element (15) is arranged.

5. Syringe (2) according to claim 3, **characterised in that** the sleeve element (6) has a distal opening (14), the internal diameter (14a) of the distal opening (14) being larger, at least in portions, than the external diameter (13d) of the piercing means protection device (13), such that the piercing means protection device (13) can be arranged inside the sleeve element (6).

6. Syringe (2) according to either claim 3 or claim 4, **characterised in that** the piercing means protection device (13) can be brought into operative contact with the collar element (7), as a result of which the collar element (7) can be locked with respect to a rotation.

7. Syringe (2) according to claims 3-6, **characterised in that** the cap element (11) is formed integrally with the piercing means protection device (13).

8. Syringe (2) according to claims 3-6, **characterised in that** the cap element (11) has a distal opening (21), the distal opening (21) being formed as a receptacle in order to receive the piercing means protection device (13).

9. Syringe (2) according to any of the preceding claims, **characterised in that** the safety device (1) has at least one spring element (22) which is operatively connected to the syringe body (3) and counteracts the movement of the sleeve element (6) relative to the safety device (1).

10. Syringe (2) according to any of the preceding claims, **characterised in that** the at least one guide slot (10) comprises a first slot region (23) and a second slot region (24) which are separated from one another by a fictive separating line (25) extending along the axial direction (X) of the syringe body (3), it being possible for the guide projection (9) to be arranged in an initial position in the first slot region (23) and to be transferred from the first slot region (23) into the second slot region (24) into an end position by crossing the separating line (25) when a distal end (26) of the piercing means (5) is arranged at the level of the distal opening (14) of the sleeve element (6) as the syringe body (3) is moved relative to the sleeve element (6).

11. Syringe (2) according to claim 10, **characterised in that** the at least one guide projection (9) can be transferred, by means of a slot of the second slot region (24), from the second slot region (24) into an end region (27) in which a movement of the sleeve element (6) relative to the syringe body (3) substantially along the axial direction (X) is at least limited.

## Revendications

1. Seringue (2) ayant un corps de seringue (3), un moyen de piqûre (5) disposé sur la pièce d'extrémité conique distale (4) du corps de seringue (3) et un dispositif de sécurité (1) pour éviter des blessures par piqûre, comportant un élément manchon (6) s'étendant le long d'une direction axiale (X), lequel entoure au moins partiellement le moyen de piqûre (5) et le corps de seringue (3), et un élément collier (7), lequel est apte à être disposé sur une région d'extrémité distale (8) du corps de seringue (3) et arrête le dispositif de sécurité (1) sur une saillie de la pièce d'extrémité conique dans la direction axiale (X), l'élément collier (7) présentant au moins une saillie de guidage (9), laquelle s'engage dans au moins une coulisse de guidage (10) de l'élément manchon (6),
**caractérisé par le fait que**
l'élément collier (7) est disposé sur la région d'extrémité distale (8) du corps de seringue (3) en étant apte à tourner dans une direction périphérique (U) et le dispositif de sécurité (1) présente un élément capuchon (11) qui est apte à être disposé en moins par sections sur l'élément manchon (6), au moyen duquel l'élément manchon (6) est apte à être arrêté en ce qui concerne le déplacement relatif du corps de seringue (3) par rapport à l'élément manchon (6), et lequel présente un dispositif (13) de protection de moyen de piqûre, dans lequel le moyen de piqûre (5) est apte à être disposé, l'élément capuchon (11) et l'élément manchon (6) présentant des éléments d'encliquetage (15, 16) complémentaires l'un de l'autre, un élément d'encliquetage (15, 16) présentant une zone de rupture, et un retrait de l'élément capuchon (11) de l'élément manchon (6) nécessitant une rupture de la zone de rupture.

2. Seringue (2) selon la revendication 1,
**caractérisé par le fait que**
l'élément collier (7) est réalisé sensiblement en tant que cylindre à base circulaire creux (12), le cylindre à base circulaire (12) présentant une surface d'enveloppe (12a) sur laquelle ladite au moins une saillie de guidage (9) est disposée.

3. Seringue (2) selon la revendication 1,
**caractérisé par le fait qu'**
une rotation de l'élément collier (7) est provoquée par le déplacement relatif du corps de seringue (3) par rapport à l'élément manchon (6) et par le guidage de la saillie de guidage (9) dans la coulisse de guidage (10).

4. Seringue (2) selon la revendication 1,
**caractérisé par le fait que**
l'élément manchon (6) présente une région distale (19) sur laquelle au moins un élément d'encliquetage (15) est disposé.

5. Seringue (2) selon la revendication 3,
**caractérisé par le fait que**
l'élément manchon (6) présente une ouverture distale (14), le diamètre interne (14a) de l'ouverture distale (14) étant au moins par sections plus grand que le diamètre externe (13d) du dispositif (13) de protection de moyen de piqûre, de telle sorte que le dispositif (13) de protection de moyen de piqûre est apte à être disposé à l'intérieur de l'élément manchon (6).

6. Seringue (2) selon l'une des revendications 3 et 4,
**caractérisé par le fait que**
le dispositif (13) de protection de moyen de piqûre est apte à être amené en contact fonctionnel avec l'élément collier (7), ce par quoi l'élément collier (7) est apte à être arrêté en ce qui concerne une rotation.

7. Seringue (2) selon l'une des revendications 3 à 6,
**caractérisé par le fait que**
l'élément capuchon (11) est formé d'un seul tenant avec le dispositif (13) de protection de moyen de piqûre.

8. Seringue (2) selon l'une des revendications 3 à 6,
**caractérisé par le fait que**
l'élément capuchon (11) présente une ouverture distale (21), l'ouverture distale (21) étant réalisée en tant que logement pour recevoir le dispositif (13) de protection de moyen de piqûre.

9. Seringue (2) selon l'une des revendications précédentes,
**caractérisé par le fait que**
le dispositif de sécurité (1) présente au moins un élément élastique (22), qui est relié fonctionnellement au corps de seringue (3) et qui agit à l'encontre du déplacement relatif de l'élément manchon (6) par rapport au dispositif de sécurité (1).

10. Seringue (2) selon l'une des revendications précédentes,
**caractérisé par le fait que**
ladite au moins une coulisse de guidage (10) comporte une première (23) et une seconde (24) région de coulisse, lesquelles sont séparées l'une de l'autre par une ligne de séparation fictive (25) s'étendant le long de la direction axiale (X) du corps de seringue (3), la saillie de guidage (9) étant apte à être disposée dans la première région de coulisse (23) dans une position de départ et étant apte à être transférée de la première (23) dans la seconde région de coulisse (24) dans une position finale par franchissement de la ligne de séparation (25), lorsqu'une extrémité distale (26) du moyen de piqûre (5) est disposée au niveau de l'ouverture distale (14) de l'élément manchon (6) lors du déplacement relatif du corps de seringue (3) par rapport à l'élément manchon (6).

11. Seringue (1) selon la revendication 10,
**caractérisé par le fait que**
ladite au moins une saillie de guidage (9) est apte à être transférée de la seconde région de coulisse (24) au moyen d'une coulisse de la seconde région de coulisse (24) dans une région d'extrémité (27), dans laquelle un déplacement relatif de l'élément manchon (6) par rapport au corps de seringue (3) est au moins limité sensiblement le long de la direction axiale (X).
